# EUROPEAN PATENT SPECIFICATION

(11) **EP 0 464 156 B1**
(45) Date of publication and mention of the grant of the patent: **29.05.1996**
(21) Application number: 90907977.4
(22) Date of filing: 09.04.1990
(51) Int. Cl.: C07C 2/58

(54) **ISOPARAFFIN-OLEFIN ALKYLATION PROCESS**
ISOPARAFFINOLEFIN-ALKYLIERUNGSPROZESS
PROCEDE D'ALKYLATION D'UNE ISOPARAFFINE AVEC UNE OLEFINE

(30) Priority: 25.01.1990 US 470014; 25.01.1990 US 470016
(43) Date of publication of application: 08.01.1992
(73) Proprietor: MOBIL OIL CORPORATION, New York New York 10017 (US)
(72) Inventor: CHILD, Jonathan, Edward, Sewell, NJ 08080 (US); HAN, Scott, Lawrenceville, NJ 08648 (US); HUSS, Albin, Jr., Chadds Ford, PA 19317 (US); KENNEDY, Clinton, Robert, Westchester, PA 19382 (US); KEVILLE, Kathleen, Marie, Woodbury, NJ 08096 (US); KIRKER, Garry, Wayne, Sewell, NJ 08080 (US); MARLER, David, Owen, Deptford, NJ 08096 (US); TABAK, Samuel, Allen, Moorestown NJ 08057-2701 (US); THOMSON, Robert, Thomas, Voorhees, NJ 08043 (US)
(74) Representative: Colmer, Stephen Gary
(86) International application number: US9001926
(87) International publication number: WO9111412

(56) References cited:
- US-A- 3 906 054
- US-A- 4 150 062
- US-A- 4 377 721
- US-A- 4 384 161
- US-A- 4 439 409
- US-A- 4 788 370
- No further relevant documents have been disclosed.

## Description

The present invention relates to a process for the alkylation of an isoparaffin with an olefin.

As a result of the curtailment in the use of tetraethyl lead as an octane-improving additive for gasoline, not only has the production of unleaded gasoline increased but the octane number specification of all grades of gasoline have increased as well. Isoparaffin-olefin alkylation is a key route to the production of highly branched paraffin octane enhancers which are to be blended into gasolines.

Alkylation involves the addition of an alkyl group to an organic molecule. Thus, an isoparaffin can be reacted with an olefin to provide an isoparaffin of higher molecular weight. Industrially, alkylation often involves the reaction of C₂-C₅ olefins with isobutane in the presence of an acidic catalyst. In the past, alkylation processes have included the use of hydrofluoric acid or sulfuric acid as catalysts under controlled temperature conditions. Low temperatures are utilized in the sulfuric acid process to minimize the undesirable side reaction of olefin polymerization and the acid strength is generally maintained at 88-94 percent by the continuous addition of fresh acid and the continuous withdrawal of spent acid. The hydrofluoric acid process is less temperature-sensitive and the acid is easily recovered and purified. However, hydrofluoric acid and sulfuric acid alkylation processes, have inherent drawbacks including environmental concerns, acid consumption and disposal of corrosive materials. With the increasing demands for octane and increasing environmental concerns, it is desirable to develop an alkylation process based on a solid catalyst system.

Crystalline metallosilicates, or zeolites, have been widely investigated for use in the catalysis of isoparaffin alkylation. For example, U.S. Patent No. 3,251,902 describes the use of a fixed bed of ion-exchanged crystalline aluminosilicate having a reduced number of available acid sites for the liquid phase alkylation of C₄-C₂₀ branched-chain paraffins with C₂-C₁₂ olefins. The patent further discloses that the C₄-C₂₀ branched-chain paraffin should be allowed to substantially saturate the crystalline aluminosilicate before the olefin is introduced to the alkylation reactor.

U.S. Patent No. 3,549,557 describes the alkylation of isobutane with C₂-C₃ olefins using certain crystalline aluminosilicate zeolite catalysts in a fixed, moving or fluidized bed system, the olefin being preferably injected at various points in the reactor.

U.S. Patent No. 3,644,565 discloses the alkylation of a paraffin with an olefin in the presence of a catalyst comprising a Group VIII noble metal present on a crystalline aluminosilicate zeolite, the catalyst having been pretreated with hydrogen to promote selectivity.

U.S. Patent No. 3,655,813 discloses a process for alkylating C₄-C₅ isoparaffins with C₃-C₉ olefins using a crystalline aluminosilicate zeolite catalyst wherein a halide adjuvant is employed in the alkylation reactor. The isoparaffin and olefin are introduced into the alkylation reactor at specified concentrations and catalyst is continuously regenerated outside the alkylation reactor.

U.S. Patent No. 3,893,942 describes an isoparaffin alkylation process employing, as catalyst, a Group VIII metal-containing zeolite which is periodically hydrogenated with hydrogen in the gas phase to reactivate the catalyst when it has become partially deactivated.

U.S. Patent No. 3,236,671 discloses the use, in alkylation, of crystalline aluminosilicate zeolites having silica to alumina mole ratios above 3 and also discloses the use of various metals exchanged and/or impregnated on such zeolites.

U.S. Patent No. 3,624,173 discloses the use, in isoparaffin-olefin alkylation, of zeolite catalysts containing gadolinium.

U.S. Patent No. 3,738,977 discloses the alkylation of paraffins with ethylene employing a zeolite catalyst which possesses a Group VIII metal component, the catalyst having been pretreated with hydrogen.

U.S. Patent No. 3,865,894 describes the alkylation of C₄-C₆ isoparaffin with C₃-C₉ monoolefin employing a substantially anhydrous acidic zeolite, for example acidic zeolite Y (zeolite HY), and a halide adjuvant.

U.S. Patent No. 3,917,738 describes a process for alkylating an isoparaffin with an olefin using a solid, particulate catalyst capable of absorbing the olefin. The isoparaffin and the olefin are admixed to form a reactant stream in contact with catalyst particles at the upstream end of an adsorption zone after which the reactants are passed concurrently with the catalyst so that a controlled amount of olefin is adsorbed onto the catalyst before the combination of reactants and catalyst is introduced into an alkylation zone. This controlled olefin adsorption is said to prevent polymerization of the olefin during alkylation.

U.S. Patent No. 4,377,721 describes an isoparaffin-olefin alkylation-process utilizing, as catalyst, ZSM-20, preferably HZSM-20 or rare earth cation-exchanged ZSM-20.

U.S. Patent No. 4,384,161 describes a process of alkylating isoparaffins with olefins to provide alkylate employing as catalyst a large pore zeolite capable of absorbing 2,2,4-trimethylpentane, e.g., ZSM-4, ZSM-20, ZSM-3, ZSM-18, zeolite Beta, faujasite, mordenite, zeolite Y and the rare earth metal-containing forms thereof, and a Lewis acid such as boron trifluoride, antimony pentafluoride or aluminum trichloride. The use of a large pore zeolite in combination with a Lewis acid in accordance with this patent is reported to greatly increase the activity and selectivity of the zeolite thereby effecting alkylation with high olefin space velocity and low isoparaffin/olefin ratio.

In accordance with the present invention there is provided a process for alkylating an isoparaffin with an olefin, which comprises reacting the isoparaffin and the olefin in the presence of, as catalyst, a synthetic porous crystalline zeolite having an X-ray diffraction pattern including values substantially as set forth in Table 1, infra.

US-A-439409 and EP-B-398998 disclose zeolites useful in the process of the invention.

One measure of the selectivity of an alkylation catalyst is the C₉+. yield. This fraction generally results from oligomerization of the feed olefins resulting in a loss of alkylate yield, reduced alkylate quality and the possible formation of an acidic sludge fraction. The zeolite alkylation catalyst employed in the process of this invention provides reduced C₉+ yields relative to such known zeolite alkylation catalysts as zeolite HY, e.g., as disclosed in U.S. Patent No. 3,865,894 referred to above.

The alkylate produced by the process of this invention is of high quality based on both research and motor octane numbers and as such is particularly well suited for blending into the gasoline pool.

The synthetic porous crystalline zeolite used in the alkylation process of this invention has, in its calcined form, an X-ray diffraction pattern including the lines listed in Table I below:

**TABLE I**

| Interplanar d-Spacing (A) | Relative Intensity, I/Iₒx100 |
|---|---|
| 12.36 ± 0.4 | M-VS |
| 11.03 ± 0.2 | M-S |
| 8.83 ± 0.14 | M-VS |
| 6.18 ± 0.12 | M-VS |
| 6.00 ± 0.10 | W-M |
| 4.06 ± 0.07 | W-S |
| 3.91 ± 0.07 | M-VS |
| 3.42 ± 0.06 | VS |

more specifically the lines listed in Table II below:

**TABLE II**

| Interplanar d-Spacing (A) | Relative Intensity, I/Io x 100 |
|---|---|
| 30.0 ± 2.2 | W-M |
| 22.1 ± 1.3 | W |
| 12.36 ± 0.4 | M-VS |
| 11.03 ± 0.2 | M-S |
| 8.83 ± 0.14 | M-VS |
| 6.18 ± 0.12 | M-VS |
| 6.00 ± 0.10 | W-M |
| 4.06 ± 0.07 | W-S |
| 3.91 ± 0.07 | M-VS |
| 3.42 ± 0.06 | VS |

and yet more specifically the lines listed in Table III below:

**TABLE III**

| Interplanar d-Spacing (A) | Relative Intensity, I/Io x 100 |
|---|---|
| 12.36 ± 0.4 | M-VS |
| 11.03 ± 0.2 | M-S |
| 8.83 ± 0.14 | M-VS |
| 6.86 ± 0.14 | W-M |
| 6.18 ± 0.12 | M-VS |
| 6.00 ± 0.10 | W-M |
| 5.54 ± 0.10 | W-M |
| 4.92 ± 0.09 | W |
| 4.64 ± 0.08 | W |
| 4.41 ± 0.08 | W-M |
| 4.25 ± 0.08 | W |
| 4.10 ± 0.07 | W-S |
| 4.06 ± 0.07 | W-S |
| 3.91 ± 0.07 | M-VS |
| 3.75 ± 0.06 | W-M |
| 3.56 ± 0.06 | W-M |
| 3.42 ± 0.06 | VS |
| 3.30 ± 0.05 | W-M |
| 3.20 ± 0.05 | W-M |
| 3.14 ± 0.05 | W-M |
| 3.07 ± 0.05 | W |
| 2.99 ± 0.05 | W |
| 2.82 ± 0.05 | W |
| 2.78 ± 0.05 | W |
| 2.68 ± 0.05 | W |
| 2.59 ± 0.05 | W |

Most specifically, the calcined crystalline material has an X-ray diffraction pattern which includes the lines listed in Table IV below:

**TABLE IV**

| Interplanar d-Spacing (A) | Relative Intensity, I/Io x 100 |
|---|---|
| 30.0 ± 2.2 | W-M |
| 22.1 ± 1.3 | W |
| 12.36 ± 0.4 | M-VS |
| 11.03 ± 0.2 | M-S |
| 8.83 ± 0.14 | M-VS |
| 6.86 ± 0.14 | W-M |
| 6.18 ± 0.12 | M-VS |
| 6.00 ± 0.10 | W-M |
| 5.54 ± 0.10 | W-M |
| 4.92 ± 0.09 | W |
| 4.64 ± 0.08 | W |
| 4.41 ± 0.08 | W-M |
| 4.25 ± 0.08 | W |
| 4.10 ± 0.07 | W-S |
| 4.06 ± 0.07 | W-S |
| 3.91 ± 0.07 | M-VS |
| 3.75 ± 0.06 | W-M |
| 3.56 ± 0.06 | W-M |
| 3.42 ± 0.06 | VS |
| 3.30 ± 0.05 | W-M |
| 3.20 ± 0.05 | W-M |
| 3.14 ± 0.05 | W-M |
| 3.07 ± 0.05 | W |
| 2.99 ± 0.05 | W |
| 2.82 ± 0.05 | W |
| 2.78 ± 0.05 | W |
| 2.68 ± 0.05 | W |
| 2.59 ± 0.05 | W |

These values were determined by standard techniques. The radiation was the K-alpha doublet of copper and a diffractometer equipped with a scintillation counter and an associated computer was used. The peak heights, I, and the positions as a function of 2 theta, where theta is the Bragg angle, were determined using algorithms on the computer associated with the diffractometer. From these, the relative intensities, 100 I/Iₒ, where Iₒ is the intensity of the strongest line or peak, and d (obs.) the interplanar spacing in Angstroms Units (A), corresponding to the recorded lines, were determined. In Tables I-IV, the relative intensities are given in terms of the symbols W=weak, M-medium, S=strong and VS-very strong. In terms of intensities, these may be generally designated as follows:
W = 0 - 20
M = 20 - 40
S = 40 - 60
VS = 60 - 100
It should be understood that these X-ray diffraction patterns are characteristic of all species of the zeolite. The sodium form as well as other cationic forms reveal substantially the same pattern with some minor shifts in interplanar spacing and variation in relative intensity. Other minor variations can occur depending on the Y to X, e.g., silicon to aluminum mole ratio of the particular sample, as well as its degree of thermal treatment.

The zeolite employed in the present process typically has a composition involving the molar relationship:

X₂O₃:(n)YO₂,

wherein X is a trivalent element, such as aluminum, boron, iron and/or gallium, preferably aluminum, Y is a tetravalent element such as silicon and/or germanium, preferably silicon, and n is at least 10, usually from 10 to 150, more usually from 10 to 60, and even more usually from 20 to 40. In the as-synthesized form, the zeolite has a formula, on an anhydrous basis and in terms of moles of oxides per n moles of YO₂, as follows:

(0.005-0.1)Na₂O:(1-4)R:X₂O₃:nYO₂

wherein R is an organic component. The Na and R components are associated with the zeolite as a result of their presence during crystallization, and are easily removed by post-crystallization methods hereinafter more particularly described.

The zeolite employed herein preferably thermally stable and desirably exhibits a high surface area (greater than about 400 m/gm as measured by the BET [Bruenauer, Emmet and Teller] test). In addition, the zeolite normally exhibits Equilibrium Adsorption values of greater than 4.5 wt.%, usually greater than 7 wt.%, for cyclohexane vapor, greater than 10 wt.% for n-hexane vapor and preferably greater than 10 wt.% for water vapor. As is evident from the above formula, the zeolite is synthesized nearly free of Na cations and thus possesses acid catalysis activity as synthesized. It can, therefore, be used as alkylation catalyst herein without having to first undergo an exchange step. To the extent desired, however, the original sodium cations of the as-synthesized material can be replaced in accordance with techniques well known in the art, at least in part, by ion exchange with other cations. Preferred replacement cations include metal ions, hydrogen ions, hydrogen precursor, e.g., ammonium, ions, hydrogen ions, hydrogen precursor, e.g., ammonium, ions and mixtures thereof. Particularly preferred cations are those which tailor the activity of the catalyst for alkylation. These include hydrogen, rare earth metals and metals of Groups IIA, IIIA, IVA, IB, IIB, IIIB, IVB and VIII of the Periodic Table of the Elements.

Prior to its use as alkylation catalyst herein, the zeolite should be subjected to thermal treatment to remove part or all of any organic constituent present therein.

The zeolite alkylation catalyst herein can also be used in intimate combination with a hydrogenating component such as tungsten, vanadium, molybdenum, rhenium, nickel, cobalt, chromium, manganese, or a noble metal such as platinum or palladium where a hydrogenation-dehydrogenation function is to be performed. Such component can be associated chemically and/or physically with the zeolite and/or matrix with which the zeolite may be optionally composited. Thus, e.g., the hydrogenating component can be introduced into the catalyst composition by way of co-crystallization, exchanged into the composition to the extent a Group IIIA element, e.g., aluminum, is in the structure, impregnated therein or intimately physically admixed therewith. Such component can be impregnated in, or on, the zeolite such as, for example, by, in the case of platinum, treating the zeolite with a solution containing the platinum metal-containing ion. Thus, suitable platinum compounds for this purpose include chloroplatinic acid, platinous chloride and various compounds containing the platinum amine complex.

Prior to its use as alkylation catalyst in the process of this invention, the present zeolite should be at least partially dehydrated. This can be accomplished by heating the crystals to a temperature in the range of from 200°C to 595°C in an atmosphere such as air, nitrogen, etc., and at atmospheric, subatmospheric or superatmospheric pressures for a period of from between 30 minutes to 48 hours. Dehydration can also be performed at room temperature merely by placing the crystalline material in a vacuum but a longer time will be required to achieve a suitable degree of dehydration.

The zeolite employed in the present process can be prepared from a reaction mixture containing sources of alkali or alkaline earth metal (M), e.g., sodium or potassium, cation, an oxide of trivalent element X, e.g., aluminum, an oxide of tetravalent element Y, e.g., silicon, an organic (R) directing agent, hexamethyleneimine, and water, said reaction mixture having a composition, in terms of mole rations of oxides, within the following ranges:

| Reactants | Useful | Preferred |
|---|---|---|
| YO₂/X₂O₃ | 10 - 60 | 10 - 40 |
| H₂O/YO₂ | 5 - 100 | 10 - 50 |
| OH⁻/YO₂ | 0.01 - 1.0 | 0.1 - 0.5 |
| M/YO₂ | 0.01 - 2.0 | 0.1 - 1.0 |
| R/YO₂ | 0.05 - 1.0 | 0.1 - 0.5 |

In a preferred synthesis method the YO₂ reactant contains a substantial amount of solid YO₂, e.g., at least 30 wt.% solid YO₂. Where YO₂ is silica, the use of a silica source containing at least 30 wt.% solid silica, e.g., Ultrasil (a precipitated, spray dried silica containing 90 wt.% silica) or HiSil (a precipitated hydrated SiO₂ containing 87 wt.% silica, 6 wt.% free H₂O and 4.5 wt.% bound H₂O of hydration and having a particle size of 0.02 micron) favors crystal formation from the above mixture. If another source of oxide of silicon, e.g., Q-Brand (a sodium silicate comprised of 28.8 wt.% of SiO₂, 8.9 wt.% Na₂O and 62.3 wt.% H₂O) is used, crystallization may yield little of the required zeolite and impurity phases of other crystal structures, e.g., ZSM-12, may be produced. Preferably, therefore, the YO₂, e.g., silica, source contains at least 30 wt.% solid YO₂, e.g., silica, and more preferably at least 40 wt.% solid YO₂, e.g., silica.

Crystallization of the required zeolite can be carried out at either static or stirred conditions in a suitable reactor vessel such as, e.g., polypropylene jars or teflon-lined or stainless steel autoclaves. Crystallization is generally conducted at a temperature of 80 to 225°C for 25 hours to 60 days. Thereafter, the crystals are separated from the liquid and recovered.

Crystallization is facilitated by the presence of at least 0.01 percent, preferably 0.10 percent and still more preferably 1 percent, seed crystals based on the total weight of the crystalline product formed.

Prior to use in the process of the invention, the present zeolite is preferably combined with another material, i.e, a binder, which is resistant to the temperataures and other conditions employed in the isoparaffin alkylation process of this invention. Suitable binder materials include active and inactive materials and synthetic or naturally occurring zeolites as well as inorganic materials such as clays, silica and/or metal oxides such as alumina. The latter can be either naturally occurring or provided in the form of gelatinous precipitates or gels including mixtures of silica and metal oxides. Use of a binder material in conjunction with the present zeolite, i.e., combined therewith or present during its synthesis, which itself is catalytically active may change the conversion and/or selectivity of the catalyst. Inactive materials suitably serve as diluents to control the amount of conversion so that isoparaffin alkylation products can be obtained economically and in controlled fashion without having to employ other means for controlling the rate of reaction. These materials can be incorporated into naturally occurring clays, e.g., bentonite and kaolin, to improve the crush strength of the zeolite under commercial isoparaffin alkylation operating conditions. Good crush strength is an advantageous attribute for commercial use since it prevents or delays breaking down of the catalyst into powder-like materials.

Naturally occurring clays which can be composited with the present zeolite include the montmorillonite and kaolin family, which families include the subbentonites, and the kaolins commonly known as Dixie, McNamee, Georgia and Florida clays or others in which the main mineral constituent is halloysite, kaolinite, dickite, nacrite, or anauxite. Such clays can be used in the raw state as originally mined or initially subjected to calcination, acid treatment or chemical modification. Binders useful for compositing with the zeolite also include inorganic oxides, notably alumina.

Apart from or in addition to the foregoing binder materials, the present zeolite can be composited with an inorganic oxide matrix such as silica-thoria, silica-beryllia, silica-titania as well as ternary compositions such as silica-alumina-thoria, silica-alumina-zirconia, silica-alumina-magnesia and silica-magnesia-zirconia. It may also be advantageous to provide at least a part of the foregoing matrix materials in colloidal form so as to facilitate extrusion of the bound catalyst components(s).

The relative proportions of zeolite and inorganic oxide matrix can vary widely with the zeolite content ranging from 1 to 95 percent by weight and more usually, particularly when the composite is prepared in the form of beads, in the range of 2 to 80 weight precent of the composite.

The stability of the alkylation catalyst of the invention may be increased by steaming, which is conveniently effected by contacting the zeolite with, e.g., 5-100% steam at a temperature of at least 300°C (e.g., 300-650°C) for at least one hour (e.g., 1-200 hours) at a pressure of 101-2,500 kPa. In a more particular embodiment, the catalyst can be made to undergo steaming with 75-100% steam at 315-500°C and atmospheric pressure for 2-25 hours.

The alkylation catalyst employed in the process of this invention may comprise a Lewis acid promoter in addition to the zeolite described above. A Lewis acid is generally considered to be a molecule which is capable of combining with another molecule or ion by forming a covalent chemical bond with two electrons from the second molecule or ion, which is to say, a Lewis acid is an electron acceptor. Examples of Lewis acids include boron trifluoride (BF₃), antimony pentafluoride (SbF₅) and aluminum chloride (AlCl₃). The present invention contemplates the use of these and other Lewis acids including those disclosed in "Friedel-Crafts and Related Reactions", Interscience Publishers, Chapters III and IV (1963). BF₃ is a preferred Lewis acid for use in the alkylation process of this invention. In the case of BF₃, this promoter is preferably present in the alkylation zone in an amount which exceeds that required to saturate the zeolite catalyst component considered not only as the zeolite per se but as any other material, e.g., binder or matrix material, which might be associated therewith.

The operating temperature of the alkylation process herein can extend over a broad range, e.g., from -40 to 400°C, with lower temperatures being used when Lewis acid promoter is present. Thus with a Lewis acid promoter the temperature will generally be from -40 to 250°C and preferably -20 to 100°C. When no Lewis acid promoter is employed, the temperature will generally be from -25 to 400°C, and preferably 75 to 200°C. The practical upper operating temperature will often be dictated by the need to avoid an undue occurrence of undesirable side reactions.

The pressures employed in the present process can extend over a wide range, e.g., from subatmospheric to 34580 kPa (5000 psig), preferably from 100 to 7000 kPa (1 atomsphere to 1000 psig).

The amount of zeolite used in the present alkylation process can be varied over relatively wide limits. In general, the amount of zeolite as measured by the weight hourly space velocity (WHSV) of the olefin can range from 0.01 to 100, preferably from 0.1 to 20.

The isoparaffin reactant used in the present alkylation process generally possesses up to 20 carbon atoms and preferably 4 to 8 carbon atoms, such as, for example, isobutane, 3-methylhexane, 2-methylbutane, 2,3-dimethylbutane and 2,4-dimethylhexane.

The olefin reactant employed herein generally contains from 2 to 12 carbon atoms. Representative examples are ethylene, propylene, butene-1, butene-2, isobutylene, pentenes, hexenes, heptenes and octenes. Particlarly preferred are C₃ and C₄ olefins and mixtures thereof.

In general, the mole ratio of total isoparaffin to total olefin alkylating agent in the combined hydrocarbon feed can be from 0.5:1 to 500:1, preferably from 3:1 to 50:1. The isoparaffin and/or olefin reactants can be in either the vapor phase or the liquid phase and can be neat, i.e., free from intentional admixture of dilution with other material, or the reactants can be brought into contact with the catalyst composition with the aid of carrier gases or diluents such as, for example, nitrogen.

The reactants can be introduced to the alkylation reaction zone together with one or more other materials which serve to enhance the overall conversion operation. Thus, for example, relatively small quantities of hydrogen and/or hydrogen donors can be present in the reaction zone to suppress catalyst aging. Water and/or materials such as alcohols which provide water under the alkylation conditions selected can also be introduced into the reaction zone for this purpose. Oxygen and/or other materials which tend to suppress oligomerization of the olefin feed can also be present.

Where water is to be introduced into the alkylation reactor, it is conveniently co-fed at a rate of 0.1ppmw to 1 wt.%, preferably 0.1ppmw to 500ppmw, based upon the total hydrocarbon feed rate. Alternatively, water can be preintroduced into the zeolite catalyst, conveniently in an amount ranging from 0.5 to 25%, preferably 1-10%, by weight of the catalyst.

The alkylation process of the present invention can be carried out as a batch-type, semi-continuous or continuous operation utilizing a fixed or moving bed of the zeolite catalyst component. A preferred embodiment entails use of a catalyst zone wherein the hydrocarbon charge is passed concurrently or countercurrently through a moving bed of the zeolite catalyst. The latter, after use, is conducted to a regeneration zone where coke is removed, e.g., by burning in an oxygen-containing atmosphere (such as air) at elevated temperature or by extracting with a solvent, after which the regenerated catalyst is recycled to the conversion zone for further contact with the organic reactants.

The invention will now be more particularly described with reference to the Examples and the accompanying drawings, in which:

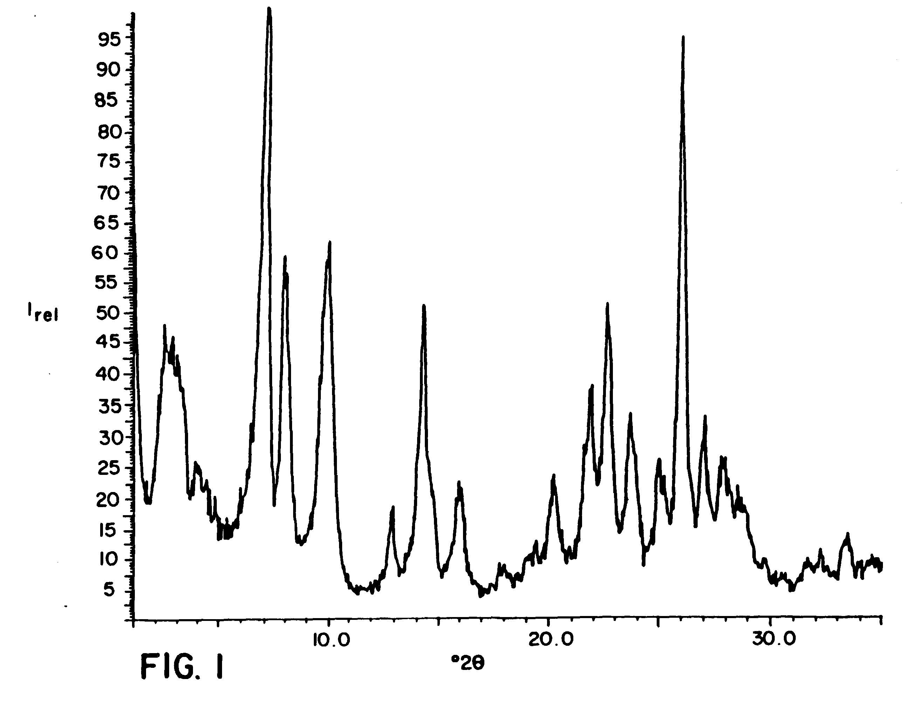

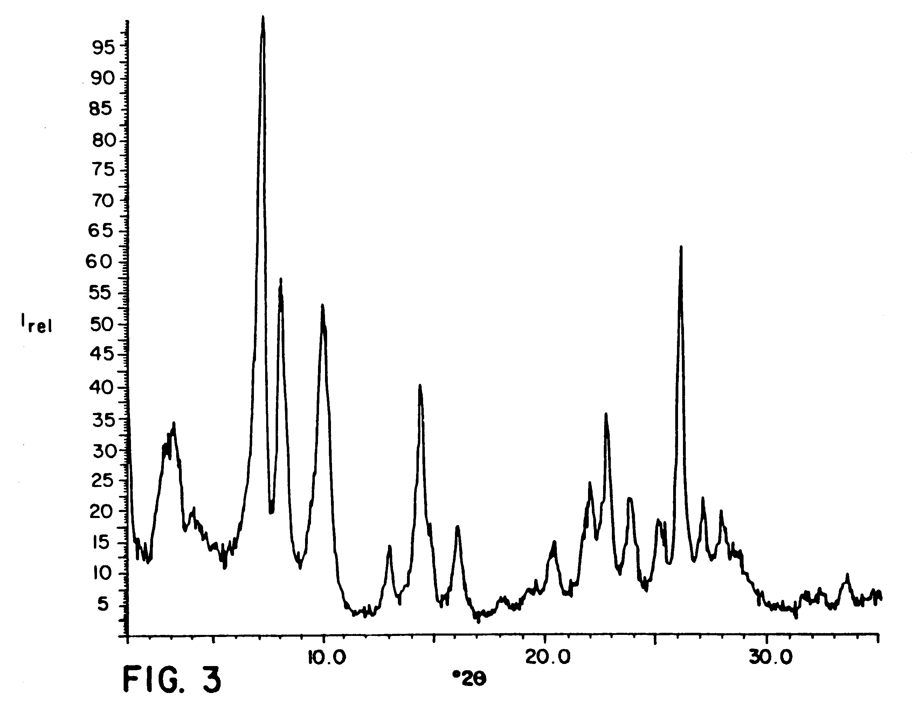

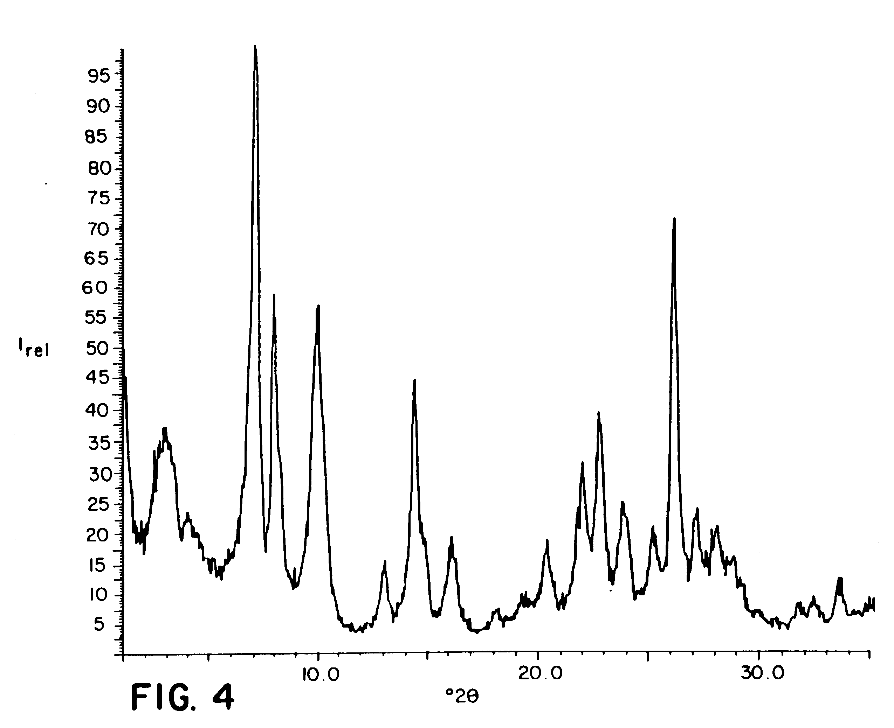

Figures 1-5 are X-ray diffraction patterns of the calcined crystalline material products of Examples 1, 3, 4, 5 and 7, respectively.

In the Examples, whenever sorption data are set forth for comparison of sorptive capacities for water, cyclohexane and/or n-hexane, they were Equilibrium Adsorption values determined as follows:

A weighed sample of the calcined adsorbent was contacted with the desired pure adsorbate vapor in an adsorption chamber, evacuated to less than 1 mm Hg and contacted with 1.6 kPa (12 Torr) of water vapor or 5.3 kPa (40 Torr) of n-hexane or 5.3 kPa (40 Torr) of cyclohexane vapor, pressures less than the vapor-liquid equilibrium pressure of the respective adsorbate at 90°C. The pressure was kept constant (within about ± 0.5 mm Hg) by addition of adsorbate vapor controlled by a manostat during the adsorption period, which did not exceed about 8 hours. As adsorbate was adsorbed by the crystalline zeolite, the decrease in pressure caused the manostat to open a valve which admitted more adsorbate vapor to the chamber to restore the above control pressures. Sorption was complete when the pressure change was not sufficient to activate the manostat. The increase in weight was calculated as the adsorption capacity of the sample in g/100 g of calcined adsorbant. The zeolite employed herein always exhibits Equilibrium Adsorption values of greater than 10 wt.% for water vapor, greater than 4.5 wt.%, usually greater than 7 wt.% for cyclohexane vapor and greater than 10 wt.% for n-hexane vapor.

When Alpha Value is examined, it is noted that the Alpha Value is an approximate indication of the catalytic cracking activity of the catalyst compared to a standard catalyst and it gives the relative rate constant (rate of normal hexane conversion per volume of catalyst per unit time). It is based on the activity of a highly active silica-alumina cracking catalyst taken as an Alpha of 1 (Rate Constant = 0.016 sec⁻¹). The Alpha Test which is used herein is described in J. Catalysis, 61, pp. 390-396 (1980). It is noted that intrinsic rate constants for many acid-catalyzed reactions are proportional to the Alpha Value for a particular crystalline silicate catalyst, i.e., the rates for toluene disproportionation, xylene isomerization, alkene conversion and methanol conversion (see "The Active Side of Acidic Aluminosilicate Catalysts," Nature, Vol. 309, No. 5969, pp. 589-591, 14 June 1984.)

### EXAMPLE 1

1 part of sodium aluminate (43.5% Al₂O₃, 32.2% Na₂O, 25.6% H₂O) was dissolved in a solution containing 1 part of 50% NaOH solution and 103.13 parts H₂O. To this was added 4.50 parts hexamethyleneimine. The resulting solution was added to 8.55 parts of Ultrasil, a precipitated, spray-dried silica (about 90% SiO₂).

The reaction mixture had the following composition, in mole ratios:

SiO₂/Al₂O₃ = 30.0

OH⁻/SiO₂ = 0.18

H₂O/SiO₂ = 44.9

Na/SiO₂ = 0.18

R/SiO₂ = 0.35

where R is hexamethyleneimine.

The mixture was crystallized in a stainless steel reactor, with stirring, at 150°C for 7 days. The crystalline product was filtered, washed with water and dried at 120°C. After a 20 hour calcination at 538°C, the X-ray diffraction pattern contained the major lines listed in Table V. Figure 1 shows the X-ray diffraction pattern of the calcined product. The sorption capacities of the calcined material were measured to be:

| | |
|---|---|
| H₂O | 15.2 wt.% |
| Cyclohexane | 14.6 wt.% |
| n-Hexane | 16.7 wt.% |

The surface area of the zeolite was measured to be 494 m/g.

The chemical composition of the uncalcined material was determined to be as follows:

| Component | wt.% |
|---|---|
| SiO₂ | 66.9 |
| Al₂O₃ | 5.40 |
| Na | 0.03 |
| N | 2.27 |
| Ash | 76.3 |
| SiO₂/Al₂O₃, mole ratio | 21.1 |

**TABLE V**

| Degrees 2-Theta | Interplanar d-Spacing (A) | I/Iₒ |
|---|---|---|
| 2.80 | 31.55 | 25 |
| 4.02 | 21.98 | 10 |
| 7.10 | 12.45 | 96 |
| 7.95 | 11.12 | 47 |
| 10.00 | 8.85 | 51 |
| 12.90 | 6.86 | 11 |
| 14.34 | 6.18 | 42 |
| 14.72 | 6.02 | 15 |
| 15.90 | 5.57 | 20 |
| 17.81 | 4.98 | 5 |
| 20.20 | 4.40 | 20 |
| 20.91 | 4.25 | 5 |
| 21.59 | 4.12 | 20 |
| 21.92 | 4.06 | 13 |
| 22.67 | 3.92 | 30 |
| 23.70 | 3.75 | 13 |
| 24.97 | 3.57 | 15 |
| 25.01 | 3.56 | 20 |
| 26.00 | 3.43 | 100 |
| 26.69 | 3.31 | 14 |
| 27.75 | 3.21 | 15 |
| 28.52 | 3.13 | 10 |
| 29.01 | 3.08 | 5 |
| 29.71 | 3.01 | 5 |
| 31.61 | 2.830 | 5 |
| 32.21 | 2.779 | 5 |
| 33.35 | 2.687 | 5 |
| 34.61 | 2.592 | 5 |

### EXAMPLE 2

A portion of the calcined crystalline product of Example 1 was tested in the Alpha Test and was found to have an Alpha Value of 224.

### EXAMPLES 3-5

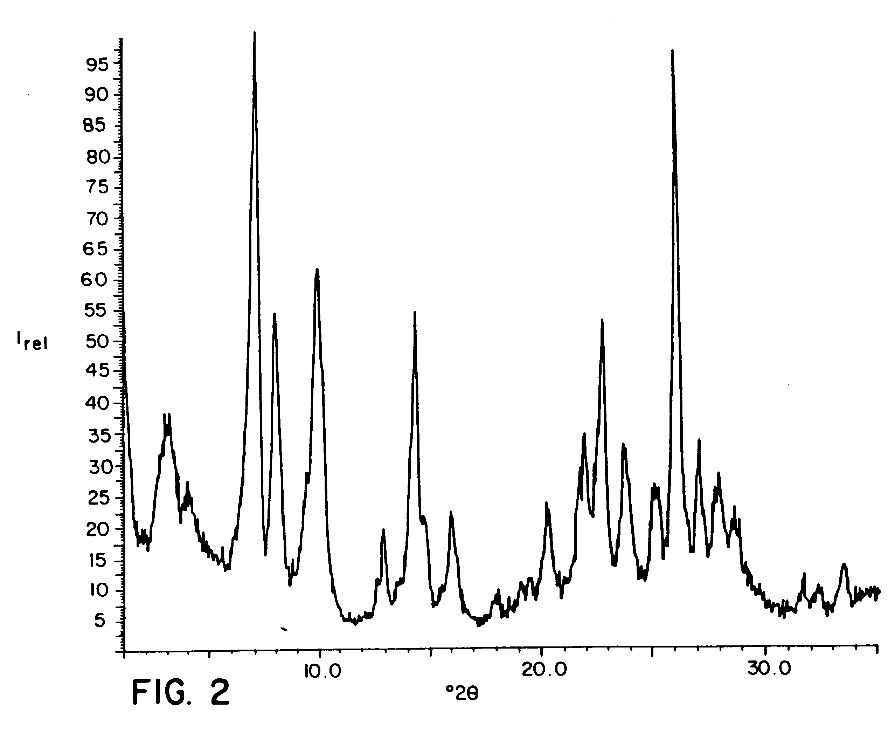

Three separate synthesis reaction mixtures were prepared with compositions indicated in Table VI. The mixtures were prepared with sodium aluminate, sodium hydroxide, Ultrasil, hexamethyleneimine (R) and water. The mixtures were maintained at 150°C, 143°C and 150°C, respectively, for 7, 8 and 6 days respectively in stainless steel autoclaves at autogenous pressure. Solids were separated from any unreacted components by filtration and then water washed, followed by drying at 120°C. The product crystals were subjected to X-ray diffraction, sorption, surface area and chemical analyses. The results of the sorption, surface area and chemical analyses are presented in Table VI and the X-ray diffraction patterns are presented in Figures 2, 3 and 4, respectively. The sorption and surface area measurements were of the calcined product.

**TABLE VI**

| Example | 3 | 4 | 5 |
|---|---|---|---|
| Synthesis Mixture, mole ratios | | | |
| SiO₂/Al₂O₃ | 30.0 | 30.0 | 30.0 |
| OH⁻/SiO₂ | 0.18 | 0.18 | 0.18 |
| H₂O/SiO₂ | 19.4 | 19.4 | 44.9 |
| Na/SiO₂ | 0.18 | 0.18 | 0.18 |
| R/SiO₂ | 0.35 | 0.35 | 0.35 |
| | | | |
| Product Composition, Wt.% | | | |
| SiO₂ | 64.3 | 68.5 | 74.5 |
| Al₂O₃ | 4.85 | 5.58 | 4.87 |
| Na | 0.08 | 0.05 | 0.01 |
| N | 2.40 | 2.33 | 2.12 |
| Ash | 77.1 | 77.3 | 78.2 |
| SiO₂/Al₂O₃, mole ratio | 22.5 | 20.9 | 26.0 |
| | | | |
| Adsorption, Wt.% | | | |
| H₂O | 14.9 | 13.6 | 14.6 |
| Cyclohexane | 12.5 | 12.2 | 13.6 |
| n-Hexane | 14.6 | 16.2 | 19.0 |
| | | | |
| Surface Area, n/g | 481 | 492 | 487 |

### EXAMPLE 6

Quantities of the calcined (538°C for 3 hours) crystalline silicate products of Examples 3, 4 and 5 were tested in the Alpha Test and found to have Alpha Values of 227, 180 and 187, respectively.

### EXAMPLE 7

To demonstrate a larger preparation of the required zeolite 1200g of hexamethyleneimine was added to a solution containing 268g of sodium aluminate, 267g of 50% Na0H solution and 11,800g of H₂O. To the combined solution was added 2,280g of Ultrasil silica. The mixture was crystallized with agitation (about 200 rpm) at 145°C in a 5 gallon reactor. Crystallization time was 59 hours. The product was water washed and dried at 120°C.

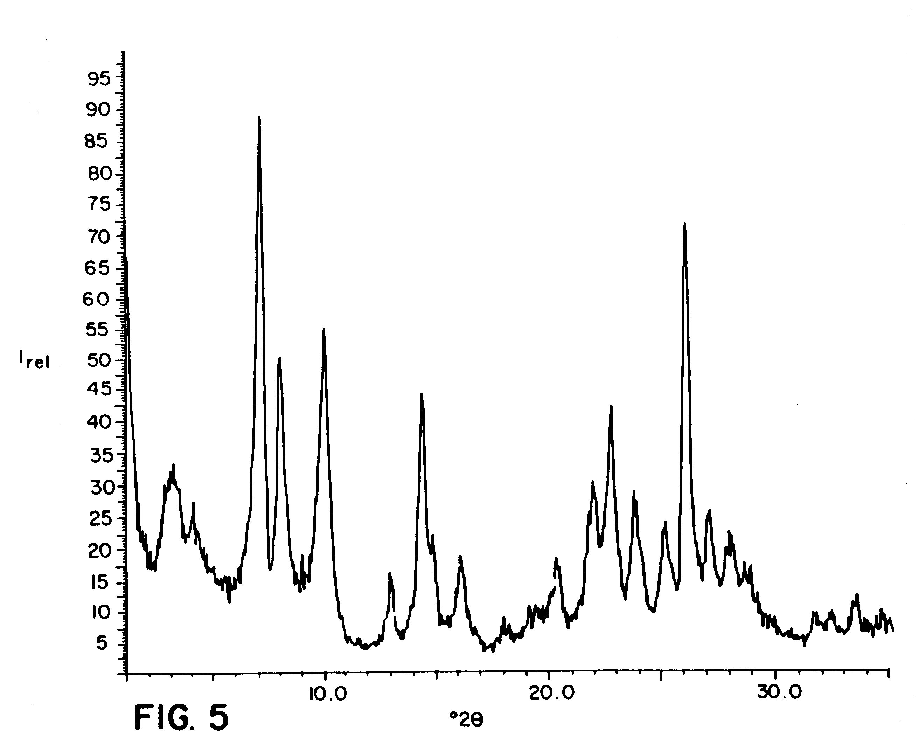

The X-ray diffraction pattern of the dried product crystals is presented in Figure 5 and demonstrates the product to he the crystalline material of this invention. Product chemical composition, surface area and adsorption analyses results were as set forth in Table VII:

**TABLE VII**

| | |
|---|---|
| Product Composition (uncalcined) | |
| C | 12.1 wt.% |
| N | 1.98 wt.% |
| Na | 640 ppm |
| Al₂O₃ | 5.0 wt.% |
| SiO₂ | 74.9 wt.% |
| SiO₂/Al₂O₃, mole ratio | 25.4 |
| | |
| Adsorption, wt.% | |
| Cyclohexane | 9.1 |
| N-Hexane | 14.9 |
| H₂O | 16.8 |
| | |
| Surface Area, m/g | 479 |

### EXAMPLE 8

25g grams of solid crystal product from Example 7 were calcined in a flowing nitrogen atmospheres at 538°C for 5 hours, followed by purging with 5% oxygen gas (balance N₂) for another 16 hours at 538°C.

Individual 3g samples of the calcined material were ion-exchanged with 100 ml of 0.1N TEABr, TPABr and LaCl₃ solution separately. Each exchange was carried out at ambient temperature for 24 hours and repeated three times. The exchanged samples were collected by filtration, water-washed to be halide-free and dried. The compositions of the exchanged samples are tabulated below demonstrating the exchange capacity of the present crystalline silicate for different ions.

| Exchange Ions Ionic Composition, wt.% | TEA | TPA | La |
|---|---|---|---|
| Na | 0.095 | 0.089 | 0.063 |
| N | 0.30 | 0.38 | 0.03 |
| C | 2.89 | 3.63 | - |
| La | - | - | 1.04 |

### EXAMPLE 9

The La-exchanged sample from Example 8 was sized to 14 to 25 mesh and then calcined in air at 538°C for 3 hours. The calcined material had an Alpha Value of 173.

### EXAMPLE 10

The calcined sample La-exchanged material from Example 9 was severely steamed at 649°C in 100% steam for 2 hours. The steamed sample had an Alpha Value of 22, demonstrating that the zeolite had very good stability under severe hydrothermal treatment.

### EXAMPLE 11

This example illustrates the preparation of the present zeolite where X in the general formula, supra, is boron. Boric acid, 2.59 parts, was added to a solution containing 1 part of 45% KOH solution and 42.96 parts H₂O. To this was added 8.56 parts of Ultrasil silica, and the mixture was thoroughly homogenized. A 3.88 parts quantity of hexamethyleneimine was added to the mixture.

The reaction mixture had the following composition in mole ratios:

SiO₂/B₂O₃ = 6.1

OH⁻/SiO₂ = 0.06

H₂O/SiO₂ = 19.0

K/SiO₂ = 0.06

R/SiO₂ = 0.30

where R is hexamethyleneimine.

The mixture was crystallized in a stainless steel reactor, with agitation, at 150°C for 8 days. The crystalline product was filtered, washed with water and dried at 120°C. A portion of the product was calcined for 6 hours at 540°C and found to have the following sorption capacities:

| | |
|---|---|
| H₂O | 11.7 wt% |
| Cyclohexane | 7.5 wt% |
| n-Hexane | 11.4 wt% |

The surface area of the calcined crystalline material was measured (BET) to be 405 m/g.

The chemical composition of the uncalcined material was determined to be as follows:

| | |
|---|---|
| N | 1.94 wt.% |
| Na | 175 ppm |
| K | 0.60 wt.% |
| Boron | 1.04 wt.% |
| Al₂O₃ | 920 ppm |
| SiO₂ | 75.9 wt.% |
| Ash | 74.11 wt.% |
| SiO₂/Al₂O₃, molar ratio | 1406 |
| SiO₂/(Al+B)₂O₃, molar ratio | 25.8 |

### EXAMPLE 12

A portion of the calcined crystalline product of Example 11 was treated with NH₄Cl and again calcined. The final crystalline product was tested in the Alpha test and found to have an Alpha Value of 1.

### EXAMPLE 13

This example illustrates another preparation of the zeolite in which X of the general formula, supra, is boron. Boric acid, 2.23 parts, was added to a solution of 1 part of 50% NaOH solution and 73.89 parts H₂O. To this solution was added 15.29 parts of Hisil silica followed by 6.69 parts of hexamethyleneimine. the reaction mixture had the following composition in mole ratios:

SiO₂/B₂O₃ = 12.3

OH⁻/SiO₂ = 0.056

H₂O/SiO₂ = 18.6

K/SiO₂ = 0.056

R/SiO₂ = 0.30

where R is hexamethyleneimine.

The mixture was crystallized in a stainless steel reactor, with agitation, at 300°C for 9 days. The crystalline product was filtered, washed with water and dried at 120°C. The sorption capacities of the calcined material (6 hours at 540°C) were measured.

| | |
|---|---|
| H₂O | 14.4 wt.% |
| Cyclohexane | 4.6 wt.% |
| n-Hexane | 14.0 wt.% |

The surface area of the calcined crystalline material was measured to be 438m/g.

The chemical composition of the uncalcined material was determined to be as follows:

| Component | Wt.% |
|---|---|
| N | 2.48 |
| Na | 0.06 |
| Boron | 0.83 |
| Al₂O₃ | 0.50 |
| SiO₂ | 73.4 |
| SiO₂/Al₂O₃, molar ratio | 249 |
| SiO₂/(Al+B)₂O₃, molar ratio | 28.2 |

### EXAMPLE 14

A portion of the calcined crystalline product of Example 13 was tested in the Alpha Test and found to have an Alpha Value of 5.

### EXAMPLE 15

This example compares the catalytic performance of the zeolite of the invention with that of zeolite HY for the alkylation of isobutane with 2-butene.

### A. Preparation of the Zeolite of the Invention

The zeolite of the invention was produced by adding 4.50 parts of hexamethyleneimine to a mixture containing 1.01 parts sodium aluminate, 1.00 part 50% NaOH, 8.56 parts Ultrasil, VN3 and 44.29 parts deionized H₂O. The reaction mixture was heated to 143°C (290°F) and stirred in an autoclave at that temperature for crystallization. After full crystallinity was achieved, the majority of the hexamethyleneimine was removed from the autoclave by controlled distillation and the zeolite crystals were separated from the remaining liquid by filtration, washed with deionized H₂O, exchanged with ammonium and dried. A portion of this zeolite was further exchanged with aqueous solution of ammonium nitrate. The material was then dried overnight at 120°C (250°F), calcined at 480°C (900°F) for three hours in 3v/v/min N₂, then treated with 50 vol.% air/50 vol.% N₂ at 3v/v/min, also at 480°C (900°F) for one hour. The calcination was completed by raising the temperature to 540°C (1000°F) at 3°C (5°F)/min and finally switching to 100% air (3v/v/min) and holding at this temperature for six hours. The resultant zeolite had an alpha activity of 323, a surface area of 455 m/g and contained 28 ppm sodium.

### B. Preparation of Zeolite HY

The HY catalyst was prepared by exchanging 60g of NaY with 1N NH₄NO₃ for one hour at room temperature. The catalyst was filtered, washed, and the exchange procedure was repeated. The ammonium exchanged Y zeolite was calcined in air for three hours at 540°C (1000°F). The final material had an alpha activity of 61, a surface area of 721 m/g, and contained 3.0 wt.% sodium.

### C. Alkylation of Isobutane With 2-Butene

To assess the catalytic properties of the above zeolites, separate alkylation runs were carried out batch-wise in an autoclave. The start-up procedure included the charging of 10 grams of catalyst into the reactor followed by sealing of the vessel. Approximately 350 grams of isobutane/2-butene were then introduced into the autoclave and the slurry was stirred during the subsequent nitrogen pressure test. To commence the reaction, nitrogen pressure was lowered to 1135 kPa (150 psig), and the system heated at a rate of 3°C (5°F)/minute. The final reaction conditions in each run were a temperature of 120°C (250°F), autogeneous pressure 3410 kPa or (480 psig), and a stirring speed of 500 rpm.

The results of the alkylation runs are set forth in Table VIII as follows:

**TABLE VIII**

| Alkylation Conditions | | | | |
|---|---|---|---|---|
| Catalyst | Invention | Invention | HY | HY |
| iC₄/2-C₄ Mole Ratio | 50 | 10 | 50 | 10 |
| Hours on Stream | 76 | 49 | 28 | 46 |
| | | | | |
| Conversion (%) | 95 | 73 | 99 | 85 |
| | | | | |

| Product Distribution | | | | |
|---|---|---|---|---|
| C₅ | 1.5 | 0.6 | 1.8 | 1.7 |
| C₆ | 4.9 | 3.4 | 5.0 | 3.3 |
| C₇ | 3.9 | 2.2 | 7.1 | 5.7 |
| C₈ | 74.4 | 63.1 | 43.0 | 36.0 |
| C₉+ | 15.4 | 30.8 | 43.2 | 53.3 |
| | | | | |

| Distribution of C₈ Products | | | | |
|---|---|---|---|---|
| 2,2,4-trimethylpropanol | 4.4 | 1.0 | 8.1 | 2.9 |
| 2,3,3-trimethylpropane | 43.1 | 31.8 | 18.3 | 8.4 |
| 2,3,4-trimethylpropane | 34.5 | 21.7 | 14.2 | 7.7 |
| | | | | |
| Dimethylhexanes | 16.3 | 25.3 | 56.6 | 69.7 |
| Other product(s) | 1.8 | 20.3 | 2.8 | 11.2 |
| Trimethylpropanes/Dimethexanes Mole Ratio | 5.0 | 2.2 | 0.7 | 0.3 |

As these data show, the use of the alkylation catalyst of the invention resulted in substantially more trimethylpropane alkylate and significantly less C₉+ products compared to the use of zeolite HY catalyst under equivalent conversion conditions.

### EXAMPLE 16

This example compares the alkylation performance of a BF₃-promoted zeolite alkylation catalyst composition in accordance with this invention and a BF₃-promoted silica alkylation catalyst composition. The catalyst of the invention was prepared by adding a 4.49 parts quantity of hexamethyleneimine to a mixture containing 1.00 parts sodium aluminate, 1.00 parts 50t NaOH, 8.54 parts Ultrasil VN3 and 44.19 parts deionized H₂O. The reaction mixture was heated to 143°C (290°F) and stirred in an autoclave at that temperature for crystallization. After full crystallinity was achieved, the majority of the hexamethyleneimine was removed from the autoclave by controlled distillation and the zeolite crystals were separated from the remaining liquid by filtration, washed with deionized H₂O and dried. The zeolite was activated by calcining N₂ at 1000°F for six hours, followed by aqueous ammonium nitrate exchange and calcining in air at 1000°F for six hours.

In separate runs, 10g of each of the foregoing catalyst compositions and 300 ml isobutane were charged to a reactor. Following cooling of the reactor contents to the desired alkylation temperature accompanied by constant stirring at 1900 rpm, BF₃ gas was introduced to the reactor at a flow rate of 3 wt.% of the total hydrocarbon feed. The feed olefin was then continuously introduced into the reactor to initiate alkylation. The mole ratio of isobutanes to total olefin was 10:1, the reaction temperatures were 0 or 20°C as indicated below, and the WHSV (based on total olefin) was 1.3. The composition of the feed is given in Table IX and results of the alkylation operations for both catalyst compositions are set forth in Table X as follows:

**TABLE IX**

| Composition of Paraffin-Olefin Feed | |
|---|---|
| Olefin | Propylene + Butenes |
| Isobutane:Olefin Mole Ratio | 12:1 |

| Feed Component | Wt.% |
|---|---|
| propylene | 3.30 |
| isobutylene | 1.24 |
| 1-butene | 1.01 |
| 2-butene | 2.14 |
| isobutane | 92.31 |

**TABLE X**

| | BF₃-Promoted SiO₂ | | BF₃-Promoted Zeolite | |
|---|---|---|---|---|
| Alkylation Temp., °C | 0 | 20 | 0 | 20 |
| C₅ + Product, wt.% | | | | |
| C₅ | 2.9 | 7.2 | 2.5 | 5.4 |
| C₆ | 3.4 | 6.9 | 3.1 | 5.4 |
| C₇ | 36.2 | 31.2 | 39.4 | 34.6 |
| C₈ | 49.9 | 39.3 | 50.4 | 47.1 |
| C₉+ | 7.6 | 15.4 | 4.6 | 7.4 |
| TMP/DMH* | 2.0 | 1.4 | 2.2 | 1.6 |

| Octanes, Raw Gasoline | | | | |
|---|---|---|---|---|
| RON + O | 93.9 | 90.7 | 95.5 | 91.5 |
| MON + O | 92.2 | 89.3 | 92.2 | 89.6 |

| | | | | |
|---|---|---|---|---|
| * TMP = trimethylpentanes DMH = dimethylhexanes | | | | |

### EXAMPLE 17

Example 16 was substantially repeated but with a feed having the composition set forth in Table XI as follows:

**TABLE XI**

| Composition of Paraffin-Olefin Feed | |
|---|---|
| Olefin | Propylene + Butenes |
| Isobutane:Olefin Mole Ratio | 5.7:1 |

| Feed Component | Wt.% |
|---|---|
| propylene | 5.60 |
| isobutylene | 2.90 |
| 1-butene | 2.00 |
| 2-butene | 4.45 |
| isobutane | 84.40 |
| n-butane | 0.65 |

The results of the alkylation operations are set forth in Table XII as follows:

**TABLE XII**

| | BF₃-Promoted SiO₂ | | BF₃-Promoted Zeolite | |
|---|---|---|---|---|
| Alkylation Temp., °C | 0 | 20 | 0 | 20 |

| C₅+ Product, wt.% | | | | |
|---|---|---|---|---|
| C₅ | 2.5 | 4.0 | 3.0 | 3.7 |
| C₆ | 2.6 | 3.5 | 3.3 | 3.4 |
| C₇ | 28.6 | 22.9 | 22.5 | 20.4 |
| C₈ | 56.8 | 56.0 | 64.7 | 63.9 |
| C₉+ | 9.4 | 13.6 | 6.4 | 8.6 |
| TMP/DMH | 2.2 | 1.6 | 2.2 | 1.6 |

| Octanes, Raw Gasoline | | | | |
|---|---|---|---|---|
| RON + O | 93.2 | 86.8 | 93.5 | 88.4 |
| MON + O | 92.1 | 88.3 | 92.0 | 88.2 |

### EXAMPLE 18

Example 16 was substantially repeated but with a feed having the composition set forth in Table XIII as follows:

**TABLE XIII**

| Composition of Paraffin-Olefin Feed | |
|---|---|
| Olefin | Propylene + Butenes |
| Isobutane:Olefin Mole Ratio | 10:1 |

| Feed Component | Wt.% |
|---|---|
| isobutylene | 5.73 |
| 1-butene | 0.26 |
| 2-butene | 2.98 |
| isobutane | 90.93 |
| n-butane | 0.10 |

The results of the alkylation operations are set forth in Table XIV as follows:

**TABLE XIV**

| | BF₃-Promoted SiO₂ | | BF₃-Promoted Zeolite | |
|---|---|---|---|---|
| Alkylation Temp., °C | 0 | 20 | 0 | 20 |

| C₅+ Product, wt.% | | | | |
|---|---|---|---|---|
| C₅ | 4.9 | 7.0 | 4.3 | 6.4 |
| C₆ | 4.5 | 4.7 | 3.4 | 4.5 |
| C₇ | 4.1 | 5.3 | 3.4 | 4.9 |
| C₈ | 77.9 | 71.9 | 83.5 | 77.7 |
| C₉+ | 8.7 | 11.2 | 5.5 | 6.6 |
| TMP/DMH | 5.1 | 2.9 | 4.9 | 2.9 |

| Octanes, Raw Gasoline | | | | |
|---|---|---|---|---|
| RON + O | 95.5 | 92.8 | 95.9 | 93.1 |
| MON + O | 93.1 | 91.7 | 94.2 | 93.1 |

### EXAMPLE 19

Example 16 was substantially repeated but with a feed having the composition set forth in Table XV as follows:

**TABLE XV**

| Composition of Paraffin-Olefin Feed | |
|---|---|
| Olefin | Propylene + Butenes |
| Isobutane:Olefin Mole Ratio | 12.9:1 |

| Feed Component | Wt.% |
|---|---|
| propylene | 3.22 |
| isobutylene | 2.74 |
| 1-butene | 0.15 |
| 2-butene | 1.24 |
| isobutane | 92.50 |
| n-butane | 0.15 |

The results of the alkylation operation are set forth in Table XVI as follows:

**TABLE XVI**

| | BF₃-Promoted SiO₂ | | BF₃-Promoted Zeolite | |
|---|---|---|---|---|
| Alkylation Temp., °C | 0 | 20 | 0 | 20 |

| C₅+ Product, wt.% | | | | |
|---|---|---|---|---|
| C₅ | 6.5 | 10.1 | 3.3 | 4.9 |
| C₆ | 6.3 | 8.8 | 3.0 | 4.3 |
| C₇ | 38.6 | 32.7 | 37.8 | 25.6 |
| C₈ | 33.7 | 29.7 | 51.4 | 59.4 |
| C₉+ | 14.8 | 18.7 | 4.6 | 5.9 |
| TMP/DMH | 4.1 | 2.1 | 5.4 | 3.0 |

| Octanes, Raw Gasoline | | | | |
|---|---|---|---|---|
| RON + O | 92.9 | 90.5 | 93.2 | 93.3 |
| MON + O | 90.7 | 89.5 | 92.8 | 91.7 |

### EXAMPLE 20

Example 16 was substantially repeated but with a feed having the composition set forth in Table XVII as follows:

**TABLE XVII**

| Composition of Paraffin-Olefin Feed | |
|---|---|
| Olefin | Butene |
| Isobutane:Olefin Mole Ratio | 9.6:1 |

| Feed Component | Wt.% |
|---|---|
| 2-butene | 9.4 |
| isobutane | 90.60 |

The results of the alkylation operation are set forth in Table XVIII as follows:

**TABLE XVIII**

| | BF₃-Promoted SiO₂ | | BF₃-Promoted Zeolite | |
|---|---|---|---|---|
| Alkylation Temp., °C | 0 | 20 | 0 | 20 |
| C₅+ Product, wt.% | | | | |
| C₅ | 2.7 | 7.1 | 1.9 | 3.6 |
| C₆ | 2.7 | 5.6 | 2.0 | 3.2 |
| C₇ | 2.8 | 6.3 | 1.9 | 3.9 |
| C₈ | 82.9 | 63.9 | 92.2 | 86.8 |
| C₉+ | 8.9 | 17.2 | 2.0 | 2.6 |
| TMP/DMH | 5.9 | 2.8 | 6.7 | 3.4 |

| Octanes, Raw Gasoline | | | | |
|---|---|---|---|---|
| RON + O | 97.3 | 93.2 | 98.2 | 94.8 |
| MON + O | 94.0 | 91.9 | 95.6 | 93.3 |

### EXAMPLES 21 - 24

The procedure of Example 16 was repeated with a BF₃-promoted zeolite alkylation catalyst of the invention (Example 21) and the results were compared with those obtained with a similar catalyst to which water had been added (Examples 22 and 23) and with a BF₃/H₂O catalyst system. The results are summarized in Table XIX.

From Table XIX, it will be seen that, at identical process conditions, the BF₃/zeolite/H₂O system was more active than the catalyst without H₂O (100 vs. 57% olefin conversion) and produced a higher quality of alkylate product (6.2 vs. 71.7 wt% C₉₊). In addition, the alkylate produced by the BF₃/zeolite/H₂O catalyst was superior (higher C₈ and lower C₉₊) to the product from the BF₃/H₂O system.

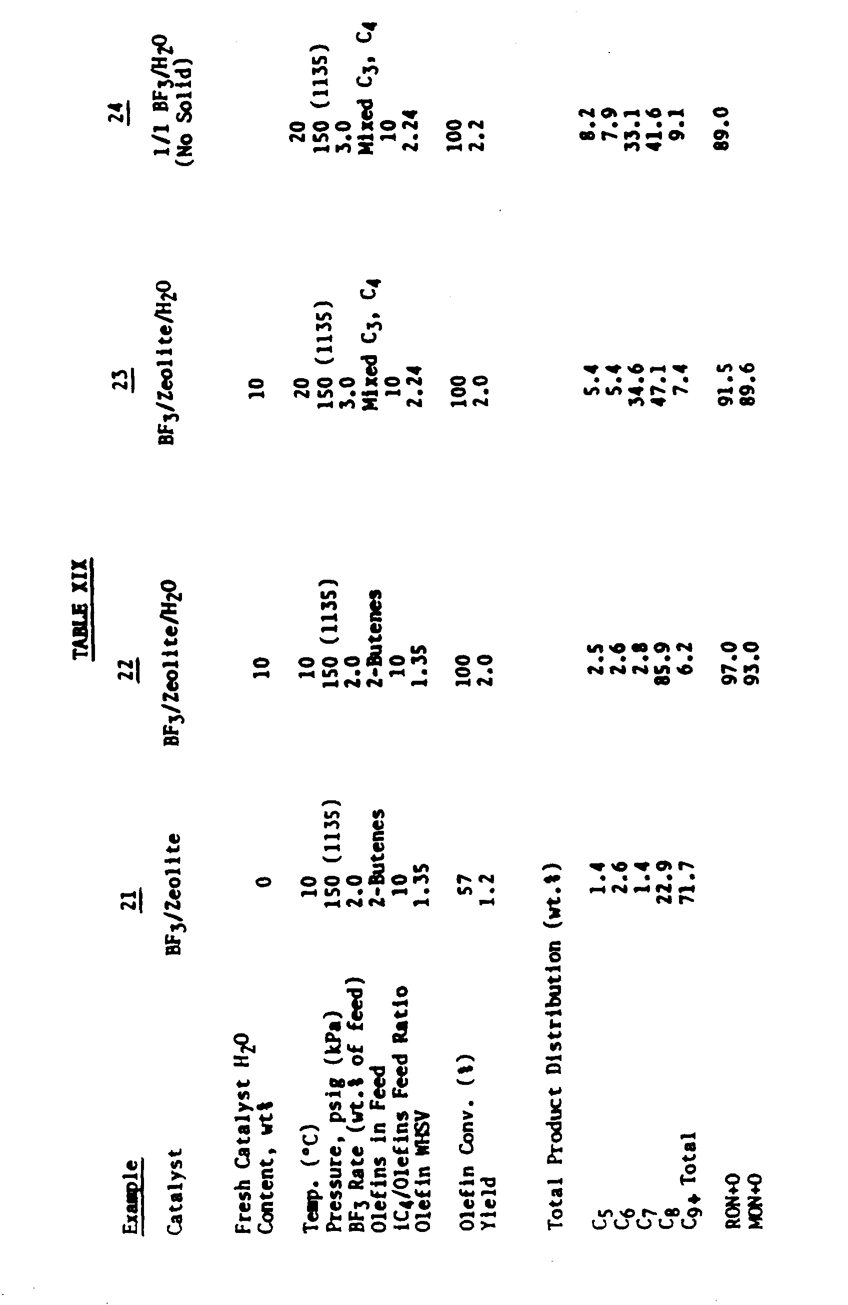

## Claims

1. A process of alkylating an isoparaffin with an olefin to provide an alkylate product which comprises reacting the isoparaffin and the olefin under alkylation conditions in the presence of, as catalyst, a zeolite having an X-ray diffraction pattern including values as set forth below:
| Interplanar d-Spacing (A) | Relative Intensity, I/Iₒx100 |
|---|---|
| 12.36 ± 0.4 | M-VS |
| 11.03 ± 0.2 | M-S |
| 8.83 ± 0.14 | M-VS |
| 6.18 ± 0.12 | M-VS |
| 6.00 ± 0.10 | W-M |
| 4.06 ± 0.07 | W-S |
| 3.91 ± 0.07 | M-VS |
| 3.42 ± 0.06 | VS |

2. The process of Claim 1, wherein the zeolite has an X-ray diffraction pattern with the lines set forth below:
| Interplanar d-Spacing (A) | Relative Intensity, I/Iₒx100 |
|---|---|
| 30.0 ± 2.2 | W-M |
| 22.1 ± 1.3 | W |
| 12.36 ± 0.4 | M-VS |
| 11.03 ± 0.2 | M-S |
| 8.83 ± 0.14 | M-VS |
| 6.18 ± 0.12 | M-VS |
| 6.00 ± 0.10 | W-M |
| 4.06 ± 0.07 | M-VS |
| 3,91 ± 0,07 | M-VS |
| 3.42 ± 0.06 | VS |

3. The process of claim 1, wherein the zeolite has an X-ray diffraction pattern with the lines set forth below:
| Interplanar d-Spacing (A) | Relative Intensity, I/Iₒx100 |
|---|---|
| 12.36 ± 0.4 | M-VS |
| 11.03 ± 0.2 | M-S |
| 8.83 ± 0.14 | M-VS |
| 6.86 ± 0.14 | W-M |
| 6.18 ± 0.12 | M-VS |
| 6.00 ± 0.10 | W-M |
| 5.54 ± 0.10 | W-M |
| 4.92 ± 0.09 | W |
| 4.64 ± 0.08 | W |
| 4.41 ± 0.08 | W-M |
| 4.25 ± 0.08 | W |
| 4.10 ± 0.07 | W-S |
| 4.06 ± 0.07 | W-S |
| 3.91 ± 0.07 | M-VS |
| 3.75 ± 0.06 | W-M |
| 3.56 ± 0.06 | W-M |
| 3.42 ± 0.06 | VS |
| 3.30 ± 0.05 | W-M |
| 3.20 ± 0.05 | W-M |
| 3.14 ± 0.05 | W-M |
| 3.07 ± 0.05 | W |
| 2.99 ± 0.05 | W |
| 2.82 ± 0.05 | W |
| 2.78 ± 0.05 | W |
| 2.68 ± 0.05 | W |
| 2.59 ± 0.05 | W |

4. The process of claim 1, wherein the zeolite has an X-ray diffraction pattern with the lines get forth below:
| Interplanar d-Spacing (A) | Relative Intensity, I/Iₒx100 |
|---|---|
| 30.0 ± 2.2 | W-M |
| 22.1 ± 1.3 | W |
| 12.36 ± 0.4 | M-VS |
| 11.03 ± 0.2 | M-S |
| 8.83 ± 0.14 | M-VS |
| 6.86 ± 0.14 | W-M |
| 6.18 ± 0.12 | M-VS |
| 6.00 ± 0.10 | W-M |
| 5.54 ± 0.10 | W-M |
| 4.92 ± 0.09 | W |
| 4.64 ± 0.08 | W |
| 4.41 ± 0.08 | W-M |
| 4.25 ± 0.08 | W |
| 4.10 ± 0.07 | W-S |
| 4.06 ± 0.07 | W-S |
| 3.91 ± 0.07 | M-VS |
| 3.75 ± 0.06 | W-M |
| 3.56 ± 0.06 | W-M |
| 3.42 ± 0.06 | VS |
| 3.30 ± 0.05 | W-M |
| 3.20 ± 0.05 | W-M |
| 3.14 ± 0.05 | W-M |
| 3.07 ± 0.05 | W |
| 2.99 ± 0.05 | W |
| 2.82 ± 0.05 | W |
| 2.78 ± 0.05 | W |
| 2.68 ± 0.05 | W |
| 2.59 ± 0.05 | W |

5. The process of Claim 1, wherein the zeolite has a composition comprising the molar relationship
X₂O₃:(n)YO₂,
wherein n is at least 10, X is a trivalent element and Y is a tetravalent element.

6. The process of Claim 5, wherein X comprises aluminum and Y comprises silicon.

7. The process of Claim 1 wherein the zeolite has equilibrium adsorption capacities of greater than 4.5 wt.% for cyclohexane vapor and greater than 10% for n-hexane vapor.

8. The process of Claim 1, wherein the isoparaffin contains from 4 to 8 carbon atoms and the olefin contains from 2 to 12 carbon atoms.

9. The process of Claim 8, wherein the isoparaffin is isobutane and the olefin is propylene and/or butene(s).

10. The process of Claim 1, wherein the mole ratio of total isoparaffin to total olefin is from 0.5:1 to 500:1.

11. The process of Claim 10, wherein the mole ratio of total isoparaffin to total olefin is from 3:1 to 50:1.

12. The process of Claim 1 wherein the alkylation reaction temperature is from -25°C to 400°C, the weight hourly space velocity of the olefin is from 0.01 to 100, and the pressure is up to 34580 kPa (5000 psig).

13. The process of Claim 1 wherein the zeolite is promoted with a Lewis acid.

14. The process of Claim 13 Wherein the Lewis acid is BF₃.

15. The process of Claim 13 or Claim 4 wherein the alkylation reaction temperature is from -40°C to 250°C, the weight hourly space velocity of the olefin is from 0.01 to 100, and the pressure is up to 34580 kPa (5000 psig).

16. The process of claim 1 wherein the alkylation reaction is conducted in the presence of water.

## Patentansprüche

1. Verfahren zur Alkylierung eines Isoparaffins mit einem Olefin zur Bildung eines Alkylatproduktes, welches umfaßt: Reaktion des Isoparaffins und des Olefins bei Alkylierungsbedingungen in Gegenwart eines Zeoliths als Katalysator mit einem Röntgenbeugungsdiagramm, das die nachfolgenden Werte umfaßt:
| d-Netzebenenabstand (A) | Relative Intensität, I/Iₒx100 |
|---|---|
| 12,36 ± 0,4 | M-VS |
| 11,03 ± 0,2 | M-S |
| 8,83 ± 0,14 | M-VS |
| 6,18 ± 0,12 | M-VS |
| 6,00 ± 0,10 | W-M |
| 4,06 ± 0,07 | W-S |
| 3,91 ± 0,07 | M-VS |
| 3,42 ± 0,06 | VS |

2. Verfahren nach Anspruch 1, wobei der Zeolith ein Röntgenbeugungsdiagramm mit den nachfolgend aufgeführten Linien aufweist:
| d-Netzebenenabstand (A) | Relative Intensität, I/Iₒx100 |
|---|---|
| 30,0 ± 2,2 | W-M |
| 22,1 ± 1,3 | W |
| 12,36 ± 0,4 | M-VS |
| 11,03 ± 0,2 | M-S |
| 8,83 ± 0,14 | M-VS |
| 6,18 ± 0,12 | M-VS |
| 6,00 ± 0,10 | W-M |
| 4,06 ± 0,07 | W-S |
| 3,91 ± 0,07 | M-VS |
| 3,42 ± 0,06 | VS |

3. Verfahren nach Anspruch 1, wobei der Zeolith ein Röntgenbeugungsdiagramm mit den nachfolgenden Linien aufweist:
| d-Netzebenenabstand (A) | Relative Intensität I/Iₒx100 |
|---|---|
| 12,36 ± 0,4 | M-VS |
| 11,03 ± 0,2 | M-S |
| 8,83 ± 0,14 | M-VS |
| 6,86 ± 0,14 | W-M |
| 6,18 ± 0,12 | M-VS |
| 6,00 ± 0,10 | W-M |
| 5,54 ± 0,10 | W-M |
| 4,92 ± 0,09 | W |
| 4,64 ± 0,08 | W |
| 4,41 ± 0,08 | W-M |
| 4,25 ± 0,08 | W |
| 4,10 ± 0,07 | W-S |
| 4,06 ± 0,07 | W-S |
| 3,91 ± 0,07 | M-VS |
| 3,75 ± 0,06 | W-M |
| 3,56 ± 0,06 | W-M |
| 3,42 ± 0,06 | VS |
| 3,30 ± 0,05 | W-M |
| 3,20 ± 0,05 | W-M |
| 3,14 ± 0,05 | W-M |
| 3,07 ± 0,05 | W |
| 2,99 ± 0,05 | W |
| 2,82 ± 0,05 | W |
| 2,78 ± 0,05 | W |
| 2,68 ± 0,05 | W |
| 2,59 ± 0,05 | W |

4. Verfahren nach Anspruch 4, wobei der Zeolith ein Röntgenbeugungsdiagramm mit den nachfolgend aufgeführten Linien aufweist:
| d-Netzebenenabstand (A) | Relative Intensität I/Iₒx100 |
|---|---|
| 30,0 ± 2,2 | W-M |
| 22,1 ± 1,3 | W |
| 12,36 ± 0,4 | M-VS |
| 11,03 ± 0,2 | M-S |
| 8,83 ± 0,14 | M-VS |
| 6,86 ± 0,14 | W-M |
| 6,18 ± 0,12 | M-VS |
| 6,00 ± 0,10 | W-M |
| 5,54 ± 0,10 | W-M |
| 4,92 ± 0,09 | W |
| 4,64 ± 0,08 | W |
| 4,41 ± 0,08 | W-M |
| 4,25 ± 0,08 | W |
| 4,10 ± 0,07 | W-S |
| 4,06 ± 0,07 | W-S |
| 3,91 ± 0,07 | M-VS |
| 3,75 ± 0,06 | W-M |
| 3,56 ± 0,06 | W-M |
| 3,42 ± 0,06 | VS |
| 3,30 ± 0,05 | W-M |
| 3,20 ± 0,05 | W-M |
| 3,14 ± 0,05 | W-M |
| 3,07 ± 0,05 | W |
| 2,99 ± 0,05 | W |
| 2,82 ± 0,05 | W |
| 2,78 ± 0,05 | W |
| 2,68 ± 0,05 | W |
| 2,59 ± 0,05 | W |

5. Verfahren nach Anspruch 1, wobei der Zeolith eine Zusammensetzung hat, die das Molverhältnis umfaßt:
X₂O₃:(n) YO₂
worin n mindestens 10 ist, X ein dreiwertiges Element und Y ein vierwertiges Element ist.

6. Verfahren nach Anspruch 5, wobei X Aluminium und Y Silicium umfaßt.

7. Verfahren nach Anspruch 1, wobei der Zeolith Gleichgewichts-Adsorptionskapazitäten von mehr als 4,5 Gew.-% für Cyclohexandampf und mehr als 10% für n-Hexandampf aufweist.

8. Verfahren nach Anspruch 1, wobei das Isoparaffin 4 bis 8 Kohlenstoffatome enthält und das Olefin 2 bis 12 Kohlenstoffatome enthält.

9. Verfahren nach Anspruch 8, wobei das Isoparaffin Isobutan und das Olefin Propylen und/oder Buten(e) ist.

10. Verfahren nach Anspruch 1, wobei das Molverhältnis des gesamten Isoparaffins zum gesamten Olefin 0,5:1 bis 500:1 beträgt.

11. Verfahren nach Anspruch 1, wobei das Molverhältnis des gesamten Isoparaffins zum gesamten Olefin 3:1 bis 50:1 beträgt.

12. Verfahren nach Anspruch 1, wobei die Temperatur der Alkylierungsreaktion -25°C bis 400°C, die stündliche Gewichts-Raum-Geschwindigkeit des Olefins 0,01 bis 100 und der Druck bis zu 34580 kPa (5000 psig) betragen.

13. Verfahren nach Anspruch 1, wobei der Zeolith mit einer Lewis-Säure gefördert wird.

14. Verfahren nach Anspruch 13, wobei die Lewis-Säure BF₃ ist.

15. Verfahren nach Anspruch 13 oder Anspruch 4, wobei die Temperatur der Alkylierungsreaktion -40°C bis 250°C, die stündliche Gewichts-Raum-Geschwindigkeit des Olefins 0,01 bis 100 und der Druck bis zu 34580 kPa (5000 psig) betragen.

16. Verfahren nach Anspruch 1, wobei die Alkylierungsreaktion in Gegenwart von Wasser erfolgt.

## Revendications

1. Un procédé d'alkylation d'une isoparaffine par une oléfine pour obtenir un alkylat, qui consiste à faire réagir l'isoparaffine avec une oléfine dans des conditions d'alkylation en présence, comme catalyseur, d'une zéolite présentant un spectre de diffraction aux rayons X comprenant les valeurs données ci-dessous:
| Espacement d-interplanaire (A) | Intensité relative, I/I_{O}x100 |
|---|---|
| 12,36 ± 0,4 | M-VS |
| 11,03 ± 0,2 | M-S |
| 8,83 ± 0,14 | M-VS |
| 6,18 ± 0,12 | M-VS |
| 6,00 ± 0,10 | W-M |
| 4,06 ± 0,07 | W-S |
| 3,91 ± 0,07 | M-VS |
| 3,42 ± 0,06 | VS |

2. Le procédé selon la revendication 1, dans lequel la zéolite présente un spectre de diffraction aux rayons X dont les raies sont données ci-dessous:
| Espacement d-interplanaire (A) | Intensité relative, I/I_{O}x100 |
|---|---|
| 30,0 ± 2,2 | W-M |
| 22,1 ± 1,3 | W |
| 12,36 ± 0,4 | M-VS |
| 11,03 ± 0,2 | M-S |
| 8,83 ± 0,14 | M-VS |
| 6,18 ± 0,12 | M-VS |
| 6,00 ± 0,10 | W-M |
| 4,06 ± 0,07 | W-S |
| 3,91 ± 0,07 | M-VS |
| 3,42 ± 0,06 | VS |

3. Le procédé selon la revendication 1, dans lequel la zéolite présente un spectre de diffraction aux rayons X dont les raies sont données ci-dessous:
| Espacement d-interplanaire (A) | Intensité relative, I/I_{O}x100 |
|---|---|
| 12,36 ± 0,4 | M-VS |
| 11,03 ± 0,2 | M-S |
| 8,83 ± 0,14 | M-VS |
| 6,86 ± 0,14 | W-M |
| 6,18 ± 0,12 | M-VS |
| 6,00 ± 0,10 | W-M |
| 5,54 ± 0,10 | W-M |
| 4,92 ± 0,09 | W |
| 4,64 ± 0,08 | W |
| 4,41 ± 0,08 | W-M |
| 4,25 ± 0,08 | W |
| 4,10 ± 0,07 | W-S |
| 4,06 ± 0,07 | W-S |
| 3,91 ± 0,07 | M-VS |
| 3,75 ± 0,06 | W-M |
| 3,56 ± 0,06 | W-M |
| 3,42 ± 0,06 | VS |
| 3,30 ± 0,05 | W-M |
| 3,20 ± 0,05 | W-M |
| 3,14 ± 0,05 | W-M |
| 3,07 ± 0,05 | W |
| 2,99 ± 0,05 | W |
| 2,82 ± 0,05 | W |
| 2,78 ± 0,05 | W |
| 2,68 ± 0,05 | W |
| 2,59 ± 0,05 | W |

4. Le procédé selon la revendication 1, dans lequel la zéolite présente un spectre de diffraction aux rayons X dont les raies sont données ci-dessous:
| Espacement d-interplanaire (A) | Intensité relative, I/I_{O}x100 |
|---|---|
| 30,0 ± 2,2 | W-M |
| 22,1 ± 1,3 | W |
| 12,36 ± 0,4 | M-VS |
| 11,03 ± 0,2 | M-S |
| 8,83 ± 0,14 | M-VS |
| 6,86 ± 0,14 | W-M |
| 6,18 ± 0,12 | M-VS |
| 6,00 ± 0,10 | W-M |
| 5,54 ± 0,10 | W-M |
| 4,92 ± 0,09 | W |
| 4,64 ± 0,08 | W |
| 4,41 ± 0,08 | W-M |
| 4,25 ± 0,08 | W |
| 4,10 ± 0,07 | W-S |
| 4,06 ± 0,07 | W-S |
| 3,91 ± 0,07 | M-VS |
| 3,75 ± 0,06 | W-M |
| 3,56 ± 0,06 | W-M |
| 3,42 ± 0,06 | VS |
| 3,30 ± 0,05 | W-M |
| 3,20 ± 0,05 | W-M |
| 3,14 ± 0,05 | W-M |
| 3,07 ± 0,05 | W |
| 2,99 ± 0,05 | W |
| 2,82 ± 0,05 | W |
| 2,78 ± 0,05 | W |
| 2,68 ± 0,05 | W |
| 2,59 ± 0,05 | W |

5. Le procédé selon la revendication 1, dans lequel la zéolite présente une composition répondant à la relation molaire:
X₂O₃:(n)YO₂
dans laquelle:
n est au moins égal à 10;
X représente un élément trivalent; et
Y représente un élément tétravalent.

6. Le procédé selon la revendication 5, dans lequel X comprend de l'aluminium et Y comprend du silicium.

7. Le procédé selon la revendication 1, dans lequel la zéolite présente des capacités d'adsorption à l'équilibre supérieures à 4,5% en poids pour la vapeur de cyclohexane et supérieures à 10% pour la vapeur de n-hexane.

8. Le procédé selon la revendication 1, dans lequel l'isoparaffine contient de 4 à 8 atomes de carbone et l'oléfine contient de 2 à 12 atomes de carbone.

9. Le procédé selon la revendication 8, dans lequel l'isoparaffine est l'isobutane et l'oléfine est le propylène et/ou un butène ou des butènes.

10. Le procédé selon la revendication 1, dans lequel le rapport molaire de l'isoparaffine totale à l'oléfine totale est compris entre 0,5/1 et 500/1.

11. Le procédé selon la revendication 1, dans lequel le rapport molaire de l'isoparaffine totale à l'oléfine totale est compris entre 3/1 et 50/1.

12. Le procédé selon la revendication 1, dans lequel la température de la réaction d'alkylation est de -25°C à 400°C, la vitesse spatiale horaire pondérale de l'oléfine est comprise entre 0,01 et 100, et la pression atteint jusqu'à 34580 kPa (5000 psig).

13. Le procédé selon la revendication 1, dans lequel la zéolite est dopée par un acide de Lewis.

14. Le procédé selon la revendication 13, dans lequel l'acide de Lewis est BF₃.

15. Le procédé selon la revendication 13 ou la revendication 4, dans lequel la température de la réaction d'alkylation est comprise entre -40°C et 250°C, la vitesse spatiale horaire pondérale de l'oléfine est comprise entre 0,01 et 100, et l'impression atteint jusqu'à 34500 kPa (5000 psig).

16. Le procédé selon la revendication 1, dans lequel la réaction d'alkylation est produite en présence d'eau.
